# EUROPÄISCHE PATENTANMELDUNG

(11) **EP 1 693 016 A1**
(43) Veröffentlichungstag der Anmeldung: **23.08.2006**
(21) Anmeldenummer: 05003324.0
(22) Anmeldetag: 16.02.2005
(51) Int. Cl.: A61B 18/20, A61N 5/06

(54) **Medizinische und/oder kosmetische Bestrahlungsvorrichtung**

(71) Anmelder: WaveLight Laser Technologie AG, 91058 Erlangen (DE)
(72) Erfinder: Vogler, Klaus, Dr., 90542 Eckental (DE); Haberer, Daniel, 91301 Forchheim (DE); Kallert, Heiko, 91448 Emskirchen (DE)
(74) Vertreter: von Hellfeld, Axel

(57) **Zusammenfassung**

Eine medizinische und/oder kosmetische Bestrahlungsvorrichtung weist mehrere LEDs (24a, 24b) auf, die bei unterschiedlichen Wellenlängen emittieren.

## Beschreibung

Die Erfindung betrifft eine medizinische und/oder kosmetische Bestrahlungsvorrichtung.

Der Stand der Technik kennt unterschiedlichste Vorrichtungen, um die menschliche Haut für Heilungszwecke und/oder kosmetische Zwecke elektromagnetischer Strahlung auszusetzen. Dabei ist bekannt, dass die Wirkungen elektromagnetischer Strahlung (nachfolgend kurz mit "Licht" bezeichnet, womit auch Strahlung mit Wellenlängen im für das menschliche Auge nicht sichtbaren Bereich erfasst sein sollen) stark von der Wellenlänge des Lichtes abhängen.

So ist zum Beispiel bekannt, dass rotes Licht in der menschlichen Haut eine verstärkte Bildung von Collagen und Procollagenen durch die Anregung von Fibroblasten bewirkt. Die Lebensdauer solcher Fibroblasten wird durch die Strahlung verlängert und es wird insgesamt eine entzündungshemmende Wirkung erzielt. Deshalb wird rotes Licht insbesondere auch zur Faltenglättung (sogenannte Skin-Rejuvenation) eingesetzt. Auch kommt Rotlicht für die Wundheilung, die sogenannten photodynamische Therapie und die Aknebekämpfung in Betracht.

Man weiß auch, dass gelbes Licht ähnlich wie Rotlicht auf die Fibroblasten wirkt und wegen vermehrter Collagenbildung die Faltenbildung reduziert. Weiterhin ist bekannt, dass unter Gelblichtbestrahlung Porphyrine (Stoffwechselprodukte von akneverursachenden Propionibakterien) freie Sauerstoffradikale bilden, wodurch die Bakterien abgetötet werden. Deshalb wird gelbes Licht bevorzugt in der Aknebehandlung eingesetzt.

Auch blaues Licht hat ähnliche, sogar noch verstärkte Wirkmechanismen in Bezug auf Aknebakterien, jedoch ist dabei die Eindringtiefe in die Haut geringer als bei gelbem Licht.

Für medizinische und/oder kosmetische Hautbehandlungen mit elektromagnetischer Strahlung werden im Stand der Technik als Lichtquellen vorrangig Laser verwendet. Dabei haben Laser bekanntlich die Vorteile einer monochromatischen Emission, einer relativ hohen Leistung und auch der Möglichkeit, einer örtlich sehr zielgenauen Lokalisierung der Bestrahlung. Diese Möglichkeiten der genauen Einstellung der Wellenlänge mit relativ geringer Bandbreite hat bei speziellen Indikationen und Behandlungen sich als besonders wirksam herausgestellt, da die Applikationsstrukturen gezielt ausgewählt und der gewünschte Effekt besonders rasch und ohne nachteilige Nebenwirkungen erreicht werden kann. Mit der hohen Leistung und Intensität der Laserstrahlung lassen sich in der Regel auch leicht Bestrahlungsintensitäten auf dem für die Behandlung vorgesehenen Hautbereich erreichen, die oberhalb der für die erwünschte Heilwirkung erforderlichen Intensitätsschwellen liegen. Diese Intensitätsschwellen werden im Stand der Technik üblicherweise als "Fluence" bezeichnet und bezeichnen eine für die Wirksamkeit der Behandlung erforderliche Leistungsdichte der Strahlung.

Allerdings sind Lasersysteme in der Regel insofern nachteilig, als sie relativ komplex aufgebaut sind und somit in der Regel für die Bedienung und Wartung ein besonders geschultes Personal erfordern. Auch sind Laser relativ teuer und stellen häufig auch Gefahrenquellen dar. Ihr Einsatz in Arztpraxen ist deshalb nur bedingt möglich und erfordert in aller Regel besondere Sicherheitsmaßnahmen.

Sollen mit Lasersystemen am selben Applikationsort Strahlungen unterschiedlicher Wellenlänge eingesetzt werden, dann erhöht sich der apparative Aufwand erheblich.

In jüngerer Zeit haben sich auf dem Markt als Alternative zu den relativ teuren Lasersystemen einfachere und damit kostengünstigere IPL-Blitzlampensysteme (IPL=Incoherent Pulsed Lamp) verbreitet. Solche multichromatischen Lichtquellen erreichen bei entsprechend starker elektrischer Einspeisung ebenfalls recht hohe Fluence-Werte, die eine Reihe von medizinischen und kosmetischen Anwendungen ermöglichen. Allerdings ist die Strahlung von IPL-Systemen wenig gerichtet, weshalb ohne besondere Zusatzausrüstungen damit eher nur eine lokal relativ unspezifische, großflächige Behandlung möglich ist. Auch emittieren IPL-Systeme in der Regel eine sehr breitbandige Strahlung, sodass dann, wenn eine Bestrahlung mit ausgewählten Wellenlängen durchgeführt werden soll, aufwendige Filteranordnungen erforderlich sind. Dabei gelingt die Monochromatisierung der Strahlung in der Regel nur ungenügend. Ein weiterer Nachteil von IPL-Systemen liegt darin, dass ein großer Teil der Emission Wärmestrahlung ist. Dies hat nicht nur Nachteile hinsichtlich der IR-Belastung des Patienten, sondern erfordert auch eine starke Kühlung der Lampensysteme selbst. Ein weiterer Nachteil der bekannten IPL-Systeme liegt darin, dass sie eine elektrische Hochspannung erforderlich machen, wodurch eine Gefahrenquelle gegeben ist, die aufwendige Sicherheitsmaßnahmen erforderlich macht. Weiterhin sind die bekannten IPL-Systeme sperrig und haben einen relativ hohen Stromverbrauch. Zwar ermöglichen IPL-Systeme durch geeignete Filterwahl die Einstellung unterschiedlicher Spektralbereiche für eine Hautbehandlung, jedoch ist dabei die Einstellung unterschiedlicher Wellenlängen zeitlich ausschließlich nacheinander möglich, d.h. ohne einen überzogenen apparativen Aufbau an Optik und Filtern ist es nicht möglich, gleichzeitig unterschiedliche Wellenlängen zur Behandlung einzusetzen.

Seit einigen Jahren stehen Festkörper-Lichtquellen mit zunehmend gesteigerten Leistungsdaten zur Verfügung, üblicherweise als LED-Lichtquellen bezeichnet (LED= Light Emitting Diode). Insbesondere stehen seit mehreren Jahren sogenannten HB LEDs (HB= High Brightness) zur Verfügung. Mit solchen LEDs sind für eine Vielzahl von medizinischen und/oder kosmetischen Anwendungen die oben erwähnten Fluence-Werte erreichbar.

Der Erfindung liegt die Aufgabe zugrunde, eine medizinische und/oder kosmetische Behandlungsvorrichtung bereitzustellen, die bei einfachem und kostengünstigem Aufbau und geringem Stromverbrauch eine hohe Vielfalt hinsichtlich der Applikationen ermöglicht.

Erreicht werden diese Ziele erfindungsgemäß mit einer medizinischen bzw. kosmetischen Bestrahlungsvorrichtung, die als Strahlungsquellen mehrere LEDs unterschiedlicher Wellenlänge aufweist, also zumindest zwei LEDs, die Strahlung unterschiedlicher Wellenlänge emittieren.

Mit einer solchen Bestrahlungsvorrichtung werden eine Reihe von Vorteilen erreicht: Zunächst kann die Monochromasie von LEDs in der oben beschriebenen Weise für medizinische und kosmetischen Anwendungen genutzt werden. Bei kompaktem Aufbau und geringem Stromverbrauch können die Abstrahlflächen der Strahlung in einfacher Weise variiert werden. Es können hohe spektrale Wirkungsgrade bei unterschiedlichen Wellenlängen ohne Notwendigkeit besonderer Filter erreicht werden. Auch ergibt sich eine hohe Betriebssicherheit bei einfacher Bedienbarkeit. Auch die Systemkosten können weitgehend reduziert werden. Darüber hinaus ermöglicht die erfindungsgemäße Vorrichtung ohne Änderung der Bestrahlungsquellen als solchen, allein durch Bereitstellung unterschiedlicher Steuerprogramme für einen Steuerungsrechner eine hohe Vielfalt an Möglichkeiten für eine zeitliche und räumliche Steuerung der auf die Haut aufzubringenden Strahlung. In der Arztpraxis oder auch außerhalb solcher können sehr unterschiedliche Behandlungsstrategien mit einem einzigen Gerät, das unterschiedlich computergesteuert ist, realisiert werden. Die bei IPL-Systemen gemäß dem Stand der Technik noch gegebenen Probleme durch Wärmestrahlung sind systembedingt vermieden.

Gemäß einer bevorzugten Ausgestaltung der Erfindung sind in einem größeren Paneel mehrere LEDs einer Wellenlänge abwechselnd mit mehreren LEDs einer anderen Wellenlänge angeordnet. Dabei können auch LEDs einer dritten Wellenlänge und auch LEDs weiterer Wellenlängen vorgesehen werden.

Gemäß einer anderen bevorzugten Ausgestaltung der Erfindung ist eine elektronische Steuerung vorgesehen, mit der die Zeitfolge der Strahlung einzelner LEDs selektiv steuerbar ist. Insbesondere können bei dieser Steuerung einer oder mehrere der folgenden Parameter wahlweise eingestellt werden: die Zeitpunkte der Strahlungsimpulse, die Längen der Strahlungsimpulse, die Intensitäten der Strahlungsimpulse, die Abstände der Strahlungsimpulse, das Verhältnis der Intensitäten von Strahlungsimpulsen unterschiedlicher Wellenlänge, und der zeitliche Verlauf der Intensität der Strahlung innerhalb der einzelnen Impulse.

Ein weiterer Vorteil der Erfindung liegt darin, dass LEDs in einem Applikationskopf eines Handgerätes angeordnet werden können. Bei dieser Variante der Erfindung kann also der Arzt bzw. das Fachpersonal das Handgerät in die Nähe der Haut des Patienten bringen, ohne dass dieser eine besondere Stellung einnehmen muss.

Eine weitere Variante der Erfindung sieht vor, dass zumindest einige der LEDs so positioniert und/oder dass vor zumindest einigen der LEDs optische Elemente so angeordnet sind, dass sich die Abstrahlkeulen der LEDs im Applikationsbereich im wesentlichen überlappen, insbesondere homogen überlappen.

Nachfolgend werden Ausführungsbeispiele der Erfindungen anhand der Zeichnung näher erläutert. Es zeigt:
- Figur 1: ein erstes Ausführungsbeispiel einer Bestrahlungsvorrichtung für medizinische und/oder kosmetische Zwecke mit einer Vielzahl von LEDs, die bei zwei unterschiedlichen Wellenlängen emittieren;
- Figur 2: eine Ausgestaltung der medizinischen und/oder kosmetischen Bestrahlungsvorrichtung als Handgerät;
- Figur 3: eine Anordnung von LEDs, zum Beispiel in einem Gerät gemäß Figur 2, derart, dass sich die jeweils emittierten Strahlungskeulen homogen überlappen;
- Figur 4: ein Ausführungsbeispiel einer zeitlichen Steuerung der von unterschiedlichen LEDs abgegebenen Strahlung;

- Figur 5: schematisch die Eindringtiefen von Strahlungen unterschiedlicher Wellenlänge in die menschliche Haut; und
- Figur 6: weitere Ausführungsbeispiele von unterschiedlichen Zeitsteuerungen einzelner LEDs mit unterschiedlichen Emissionswellenlängen.

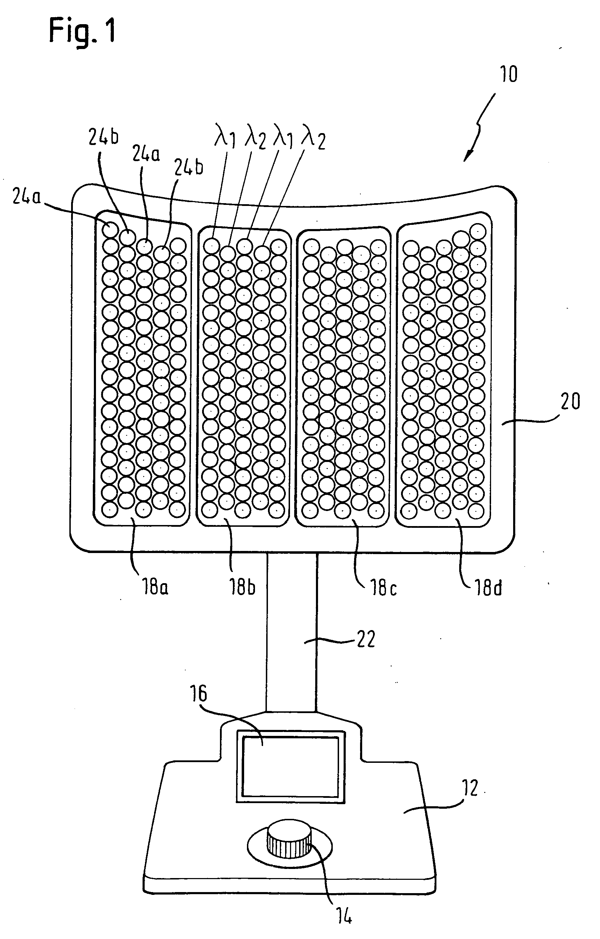

Die medizinische bzw. kosmetische Bestrahlungsvorrichtung 10 gemäß Figur 1 hat einen Fuß 12 mit einer Bedienungseinrichtung 14 und einem Anzeigefeld 16. In einer Paneele 20 sind mehrere LED-Felder 18a, 18b, 18c und 18d angeordnet. Jedes der genannten LED-Felder weist eine Vielzahl von LEDs auf. Bei diesem Ausführungsbeispiel enthält die Vorrichtung zwei verschiedene LEDs, nämlich LEDs 24a und LEDs 24b. Die LEDs 24a emittieren Licht einer ersten Wellenlänge λ₁, während die LEDs 24b Licht einer anderen Wellenlänge λ₂ emittieren. Wie dargestellt, sind die LEDs 24a bzw. 24b alternierend in Reihen angeordnet, sodass auf die zu bestrahlende Haut eines vor der Vorrichtung angeordneten Patienten Strahlungen mit zwei verschiedenen Wellenlängen gelangt, die praktisch einen einzigen örtlichen Ursprung haben, nämlich die von den LED-Feldern 18a, 18b, 18c und 18d gebildete Gesamtfläche.

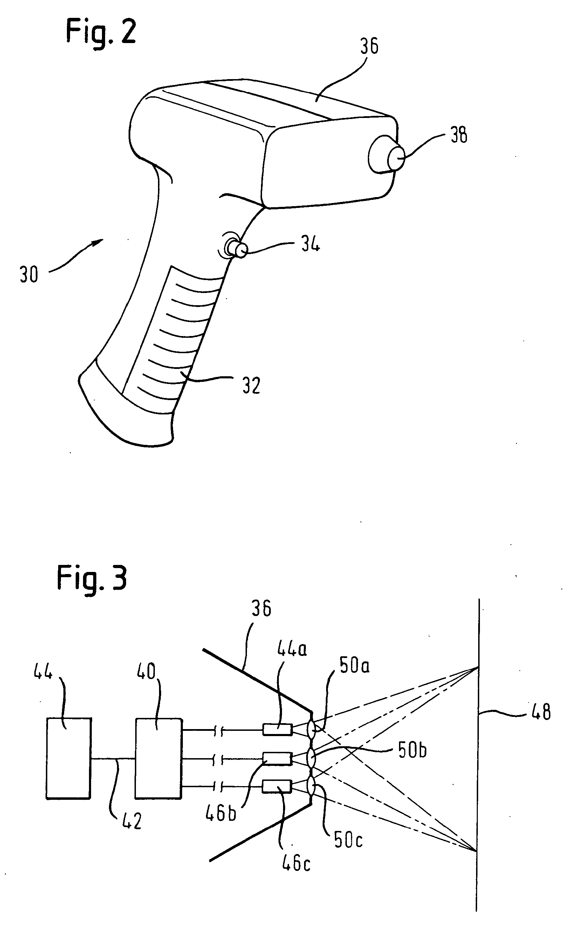

Figur 2 zeigt ein weiteres Ausführungsbeispiel einer medizinischen bzw. kosmetischen Bestrahlungsvorrichtung, die als Handgerät 30 mit einem Griff 32 ausgebildet ist. An einem Kopf 36 des Handgerätes 30 ist ein LED-Feld 38 angeordnet, das aus mehreren LEDs mit unterschiedlichen emittierten Wellenlängen bestehen kann.

Figur 3 zeigt ein Ausführungsbeispiel, wie ein LED-Feld 38 bei einer Vorrichtung gemäß Figur 2 ausgestaltet sein kann. In Figur 3 sind repräsentativ nur drei LEDs 46a, 46b und 46c dargestellt, jedoch sind eine Vielzahl weiterer LEDs analog realisierbar. Über eine elektronische Steuerung 40 werden die einzelnen LEDs 46a, 46b, 46c einzeln wahlweise angesteuert. Die elektronische Steuerung 40 ist über eine Leitung 42 mit einer Bedieneinheit 44 verbunden. Ausführungsbeispiele der zeitlichen Steuerung der einzelnen LEDs werden weiter unten näher beschrieben.

Bei der Anordnung gemäß Figur 3 sind den einzelnen LEDs 46a, 46b, 46c optische Elemente 50a, 50b bzw. 50c zugeordnet, welche die Strahlungskeulen der einzelnen LEDs gemäß Figur 3 so überlagern, dass in der Applikationsebene 48 eine gleichmä-βige, d.h. homogene Ausleuchtung erfolgt. Zusätzlich zur Wirkung der optischen Elemente 50a, b, c können die LEDs 46a, b, c auch so zueinander geneigt angeordnet werden, dass sich ihre Strahlungskeulen optimal gleichmäßig überlappen, sodass sie in der Applikationsebene 48 vom gleichen Ort auszugehen scheinen.

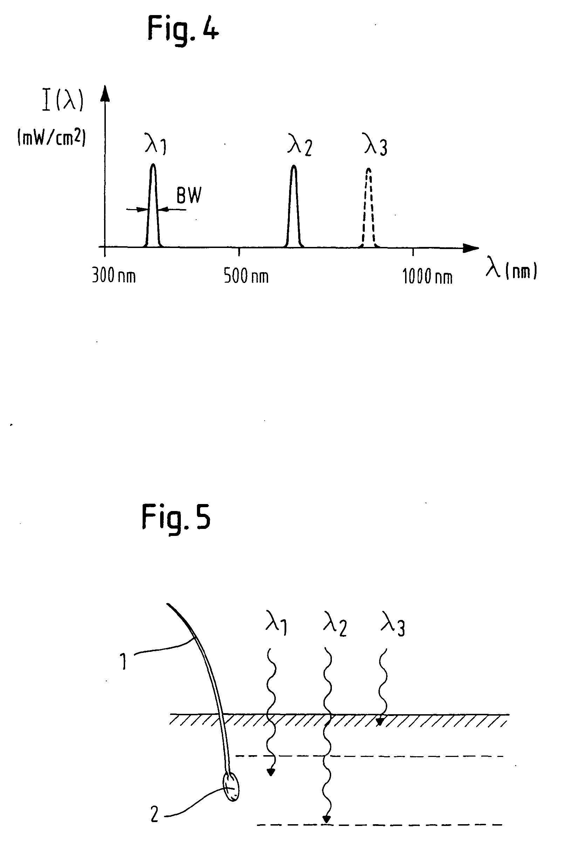

Figur 4 zeigt ein Ausführungsbeispiel für mögliche unterschiedliche Wellenlängen, die von den verschiedenen LEDs emittiert werden. LEDs sind in der Lage, spektral quasi monochromatische Emissionen abzugeben, zum Beispiel mit den Wellenlängen λ₁, λ₂ und gegebenenfalls λ₃ gemäß Figur 4. Die Bandbreite liegt dabei typischerweise im Bereich von 10 bis 20 nm. Wie dargestellt, können Wellenlängen vom UV-Bereich bis hin zum IR-Bereich eingesetzt werden. Besonders geeignet sind die eingangs angesprochenen Farbkombinationen gelb/blau zur Aknebehandlung und auch gelb/rot zur kombinierten Aknebehandlung mit Rejuvenation.

Figur 5 zeigt schematisch unterschiedliche Eindringtiefen von Strahlung mit drei verschiedenen Wellenlängen λ₁, λ₂ und λ₃. Daneben ist eine typische Tiefe eines Haares 1 mit einem Haarfollikel 2 gezeigt. Diese Darstellung illustriert den besonderen Vorteil einer Bestrahlungsvorrichtung gemäß den dargestellten Ausführungsbeispielen mit unterschiedlichen Wellenlängen, womit je nach den Zielstrukturen wahlweise unterschiedliche Eindringtiefen mit ein und derselben Vorrichtung, je nach Steuerung der einzelnen LEDs, erreicht werden können.

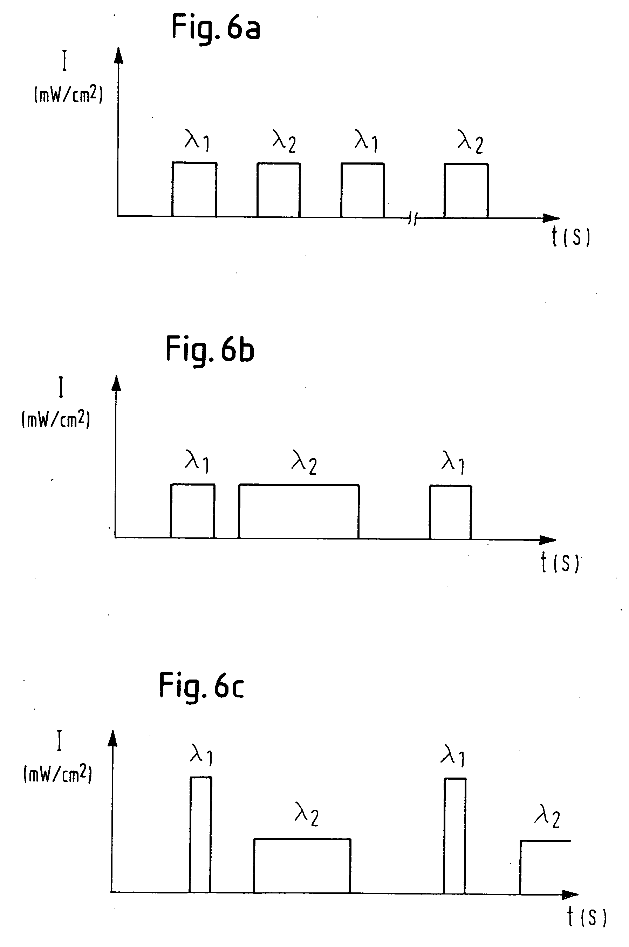

Die Figuren 6a, 6b und 6c zeigen unterschiedliche Zeitsteuerungen einzelner LEDs, wie sie beispielsweise mit einer elektronischen Steuerung 40 gemäß Figur 3 durchgeführt werden können. In den Figuren 6a, b, c sind auf der Abszisse die Strahlungsintensitäten in mW/cm² aufgetragen und auf der Ordinate die Zeit, zum Beispiel in Sekunden.

In einer einfachen Ausgestaltung können nach der Erfindung die LEDs gleichzeitig angesteuert werden, d.h. die emittierten Strahlungen unterschiedlicher Wellenlängen gelangen gleichzeitig auf die Haut (in Figur 6 nicht dargestellt). Die Erfindung ermöglicht, je nach medizinischer bzw. kosmetischer Applikation, aber einfache andere Zeitsteuerungen verschiedener Wellenlängen, ohne dass die Strahlungsquellen verändert werden. Einzustellen ist nur die Zeitsteuerung der einzelnen LEDs mittels der Steuerung 40, d.h. es liegen unterschiedliche Steuerprogramme in der Steuerung 40 vor, die vom Benutzer wahlweise mit der Bedieneinheit 44 ausgewählt und zum Einsatz gebracht werden können.

Zum Beispiel können gemäß Figur 6a die einzelnen Strahlungsimpulse unterschiedlicher Wellenlängen λ₁ bzw. λ₂ von unterschiedlichen Dioden zeitlich alternierend, aber mit gleicher Intensität abgegeben werden. Dabei können mit der Steuerung 40 sowohl die einzelnen Pulslängen, das Tastverhältnis, die Pulsintensitäten und die Abstände zwischen den Pulsen eingestellt werden. Auch eine Variation der vorstehend genannten Parameter im Verlaufe einer Behandlung kann vorab als einstellbar vorgegeben werden.

Figur 6b zeigt ein Ausführungsbeispiel, bei dem die LEDs der einen Wellenlänge λ₁ Strahlung mit einer kürzeren Pulslänge abgeben als die anderen LEDs, die bei der Wellenlänge λ₂ emittieren. Anlog lässt sich auch die Intensität wahlweise, je nach Applikation einstellen.

Figur 6c zeigt ein Ausführungsbeispiel, bei dem die Steuerung ein Programm beinhaltet, gemäß dem sowohl die Intensität als auch die Länge der einzelnen Pulse wie dargestellt variiert werden kann. Damit ist es möglich die mit der Wellenlänge λ₁ erzielte Wirkung, die bei kürzerer Pulslänge und höherer Intensität optimal ist, mit einer anderen Strahlung der Wellenlänge λ₂ zu kombinieren, die bei einer längeren Pulslänge, aber geringerer Intensität, eine optimale Wirkung entfaltet, je nach Applikation.

Die in den Figuren 6a, b und c dargestellten Ausführungsbeispiele lassen sich analog auf mehr als zwei Wellenlängen übertragen.

Besonders geeignet sind die dargestellten medizinischen bzw. kosmetischen Bestrahlungsvorrichtungen für folgende Applikationen: Skinrejuvenation, Akne-Behandlung, Wundheilung, Atopisches Exzem, Psoriasis und Vitiligo. Auch können die Geräte in phototherapeutischen Verfahren in Kombination mit Pharmaka eingesetzt werden.

Schließlich sind die beschriebenen Bestrahlungsvorrichtungen nicht nur auf therapeutische Zwecke beschränkt, sondern ermöglichen auch neue diagnostische Systeme, mit denen aufgrund der Möglichkeit, mehrere Wellenlängen zeitlich gesteuert einzustrahlen, besondere spektroskopische Nachweismöglichkeiten eröffnet werden, insbesondere der Nachweis bestimmter Absorptionsbanden und auch der Nachweis einer spezifischen Fluoreszenz.

## Patentansprüche

1. Medizinische und/oder kosmetische Bestrahlungsvorrichtung mit mehreren LEDs (18a, 18b, 18c, 18d; 24a, 24b; 46a, 46b, 46c) unterschiedlicher Wellenlänge.

2. Vorrichtung nach Anspruch 1, **dadurch gekennzeichnet, dass** mehrere LEDs (24a) einer Wellenlänge (λ₁) abwechselnd mit LEDs (24b) einer anderen Wellenlänge (λ₂) in der Vorrichtung angeordnet sind.

3. Vorrichtung nach einem der Ansprüche 1 oder 2, **gekennzeichnet durch** eine elektronische Steuerung (40), mit der die Zeitfolge der Strahlung einzelner LEDs steuerbar ist.

4. Vorrichtung nach einem der vorhergehenden Ansprüche, **dadurch gekennzeichnet, dass** die LEDs in einem Applikationskopf (36) eines Handgerätes (30) angeordnet sind.

5. Vorrichtung nach einem der vorhergehenden Ansprüche, **dadurch gekennzeichnet, dass** zumindest einige der LEDs (46a, 46b, 46c) so positioniert und/oder dass vor zumindest einigen der LEDs (46a, 46b, 46c) optische Elemente (50a, 50b, 50c) so angeordnet sind, dass sich die Abstrahlkeulen der LEDs im Applikationsbereich (48) im wesentlichen überlappen, insbesondere homogen überlappen.
